(19) Europäisches Patentamt
European Patent Office
Office européen des brevets

(11) Veröffentlichungsnummer: **0 147 735**
**B1**

(12) **EUROPÄISCHE PATENTSCHRIFT**

(45) Veröffentlichungstag der Patentschrift: **15.02.89**

(51) Int. Cl.⁴: **A 61 K 31/685**, A 61 K 45/06, A 61 K 9/14, A 61 K 9/66 // (A61K31/685, 31:54)

(21) Anmeldenummer: **84115358.8**

(22) Anmeldetag: **13.12.84**

(54) **Pharmazeutisches Präparat zur therapeutischen Behandlung von rheumatischen Erkrankungen.**

(30) Priorität: **22.12.83 DE 3346526**

(43) Veröffentlichungstag der Anmeldung: **10.07.85 Patentblatt 85/28**

(45) Bekanntmachung des Hinweises auf die Patenterteilung: **15.02.89 Patentblatt 89/7**

(84) Benannte Vertragsstaaten: **AT BE CH DE FR GB IT LI LU NL SE**

(56) Entgegenhaltungen:
**EP-A-0 054 769**
**EP-A-0 093 999**
**DE-A-2 838 851**
**DE-A-2 907 778**
**FR-A-2 343 481**
**US-A-3 822 258**

(73) Patentinhaber: **A. Nattermann & Cie. GmbH, Nattermannallee 1, D-5000 Köln 30 (DE)**

(72) Erfinder: **Wendel, Armin, Max- Ernst- Strasse 20, D-5000 Köln 40 (DE)**
Erfinder: **Wetzig, Helmut, Dr., Paulstrasse 46, D-5024 Pulheim- Sinnersdorf (DE)**
Erfinder: **Dürr, Manfred, Dr., Am Blauen Stein 10, D-5024 Pulheim- Dansweiler (DE)**
Erfinder: **Leyck, Sigurd, Dr., Am Quechenhauf 21, D-5024 Pulheim 2 (DE)**
Erfinder: **Hager, Jörg, Hermann- Josef- Schmitt- Str. 48, D-5000 Köln 30 (DE)**
Erfinder: **Ghyczy, Miklos, Dr., Am Serviesberg, D-5000 Köln 41 (DE)**

(74) Vertreter: **Sternagel, Hans- Günther, Dr., Patentanwälte Dr. Michael Hann Dr. H.- G. Sternagel Sander Aue 30, D-5060 Bergisch Gladbach 2 (DE)**

**EP 0 147 735 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Gegenstand der vorliegenden Erfindung ist ein neues, Phospholipide enthaltendes, pharmazeutisches Präparat zur therapeutischen Behandlung von rheumatischen Erkrankungen, das neben antiphlogistisch wirksamen Oxicam-Derivaten spezielles 1,2-Diacyl-glycero-3-phosphocholin im molaren Verhältnis 1 : 1 bis 1 : 20 enthält.

Zur Behandlung von rheumatischen Erkrankungen wird eine Vielzahl von nichtsteroidalen Antirheumatika eingesetzt. Eine der am häufigsten eingesetzten Substanzgruppen ist die der Oxicam-, Derivate wie z. B. Piroxicam. Die Rheumatherapie ist in der Regel eine Langzeittherapie. An Arzneimittel, deren therapeutische Anwendung in der Regel über einen langen Behandlungszeitraum notwendig ist, werden in Bezug auf die therapeutische Breite besondere Anforderungen gestellt. Als Folge der täglichen Einnahme solcher Arzneimittel ist eine hohe Belastung des Magen-Darm-Traktes als Ort der Absorption, der Leber als bevorzugtes Biotransformationsorgan, sowie der Eliminationswege unvermeidbar.

So ist bekannt, daß bei der Dauerbehandlung mit Oxicam-Derivaten z. B. gastrointestinale Störungen einschließlich Blutungen, Störungen der Hämatopoese, Kopfschmerzen, Schwindel, Leberschäden, Nierenschäden und Ödeme auftreten können. Die häufigsten Nebenwirkungen sind gastrointestinale Störungen. Die Oxicam-Derivate werden über den Weg der Leber und der Niere metabolisiert bzw. ausgeschieden. Dies kann jedoch insbesondere bei Patienten mit eingeschränkter Leber- oder Nierenfunktion zu erheblichen Nebenwirkungen führen. Bei Patienten mit vorbestehenden Leberfunktionsstörungen ist besondere Vorsicht geboten, da eine verminderte Biotransformation die Gefahr möglicher Nebenwirkungen erhöht. So mußte z. B. Sudoxicam (4-Hydroxy-2-methyl-N-(2-thiazolyl)-2H-1,2-benzthiazin-3-carboxamid-1,1-dioxid) wegen erheblicher Lebertoxizität aus der klinischen Prüfung zurückgezogen werden (Side Effects of Drugs, Annual 6; Excerpta Medica 1982, S. 103). In DE-C-2 856 333 ist beschrieben, die Magenverträglichkeit von nichtsteroidalen Antiphlogistica durch Zugabe von Phospholipiden zu verbessern, wobei das molare Verhältnis von Wirkstoff und Phospholipid bis zu 1 : 20 betragen kann. Aufgabe der vorliegenden Erfindung ist es, ein pharmazeutisches Präparat zur Behandlung rheumatischer Erkrankungen zu schaffen, das als speziellen Wirkstoff Oxicam-Derivate enthält, das eine bessere Verträglichkeit insbesondere auf die Leber zeigt, ohne die entzündungshemmende Wirksamkeit der Oxicam-Derivaten zu verändern.

Völlig überraschend wurde gefunden, daß bei niedrigen Dosierungen von Oxicam-Derivaten die in DE-C-2 856 333 beschriebene Verbesserung der Verträglichkeit im Magen-Darmtrakt, durch die Kombination mit Phospholipiden nicht in allen Fällen gegeben ist, sondern dies nur für hohe Dosierungen gilt. Kombiniert man die Oxicam-Derivate jedoch mit speziellen 1,2-Diacylglycero-3-phosphocholinen, in denen Acyl für überwiegend ungesättigte Fettsäurereste mit 16, 18 und/oder 20 Kohlenstoffatomen steht, tritt nicht nur die erwünschte Wirkung im Magen-Darmtrakt auf, sondern ist die Verträglichkeit des Präparates auch in der Leber verbessert. Die speziellen 1,2-Diacyl-glycero-3-phosphocholine weisen 75 - 86 Gew.-% ungesättigte Fettsäurereste auf. Bevorzugt sind 1,2-Diacyl-glycero-3-phosphocholine, die als Fettsäurereste Linolsäure und/oder Linolensäure- und/oder Ölsäure-und/oder Arachidonsäurereste oder Fettsäurerestgemische der folgenden Zusammensetzung enthalten:

| | | | |
|---|---|---|---|
| 10 | - | 20 | Gew.-% Palmitinsäure |
| 3 | - | 5 | Gew.-% Stearinsäure |
| 8 | - | 12 | Gew.-% Ölsäure |
| 62 | - | 69.5 | Gev.-% Linolsäure |
| 4 | - | 7 | Gew.-% Linolensäure, |

wobei die einzelnen Gehalte jeweils bei den Acylresten so ausgewählt sind, daß sie jeweils 100 Gew.-% ergeben, und der Anteil an ungesättigten Acylresten mindestens 75 Gew.-% und höchstens 86 Gew.-%, bezogen auf die Gesamtmenge der Acylreste, beträgt.

Ein Beispiel, bei dem sich die Grenzwerte jeweils auf 100 Gew.-% addieren und die Ober- und Untergrenze der ungesättigten Acylreste erreicht werden, ist folgendes:

| | | | |
|---|---|---|---|
| 20 | - | 10 | Gew.-% Palmitinsäure |
| 5 | - | 4 | Gew.-% Stearinsäure |
| 10 | - | 12 | Gew.-% Ölsäure |
| 62 | - | 68 | Gew.-% Linolsäure |
| 3 | - | 6 | Gew.-% Linolensäure |

Ganz besonders bevorzugt sind die 1,2-Diacyl-glycero-3-phosphocholine, worin der 1- und 2-Acylrest unterschiedliche Fettsäurerestgemische darstellt.

Bei diesen bevorzugten 1,2-Diacyl-glycero-3-phosphocholinen besteht der Acylrest in 1-Stellung aus folgenden Fettsäurerest-Gemischen

22 - 26 Gew.-% Palmitinsäure
6 - 9 Gew.-% Stearinsäure
8 - 12 Gew.-% Ölsäure
50 - 54 Gew.-% Linolsäure
4 - 6 Gew.-% Linolensäure

und der Acylrest in 2-Stellung besteht aus folgenden Fettsäurerest-Gemischen

1 - 2 Gew.-% Palmitinsäure
0 - 1 Gew.-% Stearinsäure
8 - 12 Gew.-% Ölsäure
78 - 85 Gew.-% Linolsäure
5 - 8 Gew.-% Linolensäure.

wobei die einzelnen Gehalte bei den Acylresten so ausgewählt sind, daß jeweils 100 Gew. erreicht werden und der Anteil an ungesättigten Acylresten mindestens 75 Gew.-% und Höchstens 86 Gew.-% beträgt.

Ein Beispiel bei dem sich die Grenzwerte in dieser Weise auf 100 Gew.-% summieren ist das Folgende

1 - Acylrest:
22 - 26 Gew.-% Palmitinsäure
7 - 9 Gew.-% Steatrinsäure
12 - 9 Gew.-% Ölsäure
54 - 50 Gew.-% Linolsäure
5 - 6 Gew.-% Linolensäure

2 - Acylrest:
1 - 3 Gew.-% Palmitinsäure
0 - 2 Gew.-% Stearinsäure
8 - 12 Gew.-% Ölsäure
85 - 75 Gew.-% Linolsäure
6 - 8 Gew.-% Linolensäure

Die speziellen 1,2-Diacyl-glycero-3-phosphocholine entsprechen der allgemeinen Formel

$$R^1OCH_2CH(OR^2)CH_2O\underset{O^-}{\underset{|}{\overset{\overset{O}{\|}}{P}}}OCH_2CH_2N^+(CH_3)_3$$

worin $R^1$ und $R^2$ gleich oder verschieden sein können und die Reste

$CH_3(CH_2)_1(CH{:}CHCH_2)_m(CH_2)_nCO-$

worin 1 = 1, 4, 7, 8 oder 10
m = 0, 1, 2, 3 oder 4
n = 2 oder 6

bedeuten, wie z. B. die folgenden Reste

$CH_3(CH_2)_{14}CO-$
$CH_3(CH_2)_{16}CO-$
$CH_3(CH_2)_7CH{:}CHCH_2(CH_2)_6CO-$
$CH_3(CH_2)_4(CH{:}CHCH_2)_2(CH_2)_6CO-$
$CH_3CH_2(CH{:}CHCH_2)_3(CH_2)_6CO-$
$CH_3(CH_2)_4(CH{:}CHCH_2)_4(CH_2)_2CO-$

Die speziellen 1,2-Diacyl-glycero-3-phosphocholine können ggfs. bis zu 20 Gew.-% weitere 1,2-Diacyl-glycero-3-phosphate bzw. deren Gemische, wie z. B. 1,2-Diacyl-glycero-3-phosphoethanolamin, 1,2-Diacyl-glycero-3-phosphoinositol, 1,2-Diacyl-glycero- 3-phosphoserin, 1,2-Diacyl-glycero-3-phosphoglycerol, insbesondere jedoch 1,2-Diacyl-glycero-3-phosphoethanolamin, enthalten.Diese weiteren Phosphocholine weisen als Acylreste die gleichen Fettsäurerestgemische auf wie die speziellen 1,2-Diacyl-glycero-3-phosphocholine. Die speziellen 1,2-Diacyl-glycero-3-phosphocholine können nach an sich bekannten Verfahren (EP 54 770, EP 54 768, EP 54 769) insbesondere aus Sojabohnen gewonnen werden.

Als Oxicam -Derivate kommen die Verbindungen der folgenden allgemeinen Formel I

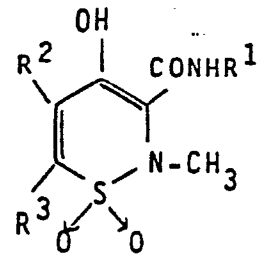

I

worin $R^1$ einen heterocyclischen Ring, wie z. B. Pyridin oder 5-Methyl-3-isoxazol bedeutet, und $R^2$ zusammen mit $R^3$ einen ankondensierten aromatischen Ring, wie z. B. den Benzol- oder Thiophenring darstellt, in Frage.

Beispiele für die Verbindungen der Formel I sind:

4-Hydroxy-2-methyl-N-(2-pyridyl)-2H-1,2-benzothiazin-3-carboxamid-1,1-dioxid (Piroxicam)

4-Hydroxy-2-methyl-N-(5-methyl-3-isoxazolyl)-2H-1,2-benzo-thiazin-3-carboxamid-1,1-dioxid (Isoxicam) 4-Hydroxy-2-methyl -N-(2-pyridyl)-2H-thieno[2,3-e]-1,2-thia-zin-3-carboxamid-1,1-dioxid (Tenoxicam)

4-Hydroxy-2-methyl-N-(2-thiazolyl)-2H-1,2-benzothiazin-3-carboxamid-1,1-dioxid (Sudoxicam).

Die Oxicam-Derivate können mit den speziellen 1,2-Diacyl-glycero-3-phosphocholinen im molaren Verhältnis von 1 : 1 bis 1 : 20 kombiniert werden, vorteilhafterweise im molaren Verhältnis 1: 1 bis 1 : 10. Eine Dosierungseinheit für die therapeutische Anwendung beim Menschen beträgt 5 - 300 mg Oxicam-Derivat kombiniert mit 10 - 500 mg 1,2-Diacyl-glycero-3-phosphochoin pro Tag, vorteilhafterweise 10 - 20 mg bei Piroxicam bzw. 100 - 300 mg bei Isoxicam kombiniert mit 50 - 250 mg 1,2-Diacyl-glycero-3-phosphocholin.

Die speziellen 1,2-Diacyl-glycero-3-phosphocholine werden, wie Ganztier-Autoradiographien zeigen, nach oraler Zufuhr schnell und dauerhaft in der Magen- und Darmschleimhaut, insbesondere jedoch in der Leber angereichert und bilden einen dauerhaften Schutz vor arzneimittelbedingten Schäden. Wie in vivo-Versuche an Ratten zeigen, können die durch die Antiphlogistika induzierten negativen Enzymveränderungen in der Leber durch den Zusatz der speziellen 1,2-Diacyl-glycero-3-phosphochline normalisiert werden.

Zur Verabreichung oder Verwendung der neuen Kombination aus speziellem 1,2-Diacyl-glycero-3-phosphocholin und Oxicam-Derivaren wird die Kombination in eine geeignete Form überführt, wie z. B. Kapseln, Lösungen, Emulsionen, Tabletten, Pulver oder Kaukapseln.

Die speziellen 1,2-Diacyl-glycero-3-phosphocholine können in Kombination mit den Oxicam-Derivaten nach an sich bekannten Verfahren in Weichgelatinekapseln oder vorteilhafter in Hartgelatinekapseln, z. B. nach dem in der DE-OS-3 022 136 beschriebenen Verfahren, ggfs. unter Zusatz geeigneter Hilfsund Füllstoffe, abgefüllt werden Die Weich- oder Hartgelatinekapseln können als Ganzes geschluckt oder auch gebaut werden.

Zur Herstellung der Hartgelatinekaukapseln werden die speziellen 1,2-Diacyl-glycero-3-phosphocholine zusammen mit den Oxicam-Derivaten mit pharmazeutisch inerten Trägerstoffen, wie z. B. Wachse, hydrierte Öle, natürliche, halbsynthetische oder synthetische Triglyceride und deren Gemische, wie Kakaobutter, sowie übliche Suppositorienmassen, z. B. auf Triglyceridbasis, wie z. B. Witepsol-Suppositorienmassen (vergl. H. P. Fiedler, Lexikon der Hilfsstoffe für Pharmazie, Kosmetik und angrenzende Gebiete, 1971, Bd. 9, S. 548-50 und 632 - 634); Fettalkohole; feste Kohlenwasserstoffe, wie Vaseline oder Paraffinsolidum; gesättigte Fettsäuren, wie Laurinsäure, Myristinsäure, Palmitinsäure, Stearinsäure; Emulgatoren, wie ethoxylierte Triglyceride, polyethoxylierte pflanzliche Öle; Fettsäurezuckerester; Silikone; Gelatine; Methylcellulose; Hydroxypropoxycellulose; hydroxypropylcellulose; Polyethylenglykole; Polyvinylpyrrolidon; Polyvinylalkohol; Polyacrylsäure und deren Salze gemischt.

Den Massen wird Ethanol in einer solchen Menge zugesetzt, daß ein konzentriertes, aber bei Raumtemperatur oder schwach erhöhter Temperatur unter Druck fließfähiges Produkt erhalten wird, d. h., daß das Produkt bei dieser Temperatur zum Transport unter Druck gerade genügend geringe oder wenig geringe Viskosität hat und auf den bekannten Abfüllanlagen mit Zusatzeinrichtung zur Flüssigabfüllung und mit erwärmbarer Abfülldüse analog der in der DE-A-3 022 136 beschriebenen Methode abgefüllt werden kann.

Die speziellen 1,2-Diacyl-glycero-3-phosphocholine können auch zusammen mit den Oxicam-Derovaten nach dem Verfahren verarbeitet werden, indem sie z. B. in die viskosen 1,2-Diacyl-glycero-3-phosphochlin-Massen vor der Abfüllung eingearbeitet werden, oder nach Abfüllung der 1,2-Diacyl- glycero-3-phosphocholine in Pulver- oder Tablettenform zudosiert werden. Zur Herstellung von Tabletten und Pulvermischungen werden die speziellen 1,2-Diacyl-glycero-3-phosphocholine in eine feste Form überführt. Dies ist wegen der Viskosität dieser Substanzen äußerst schwierig. Durch Zusatz von 2 - 10 Gew.-% Calziumchlorid werden jedoch feste Zubereitungen erhalten.

Zur Herstellung der festen Zubereitungen werden die speziellen 1,2-Diacyl-glycero-3-phosphocholine unter Zusatz üblicher Hilfsstoffe in Wasser oder einem organischen Lösungsmittel, wie z. B. Alkoholen, wie Methanol, Ethanol oder Isopropanol, in Kohlenwasserstoffen, wie Hexan, chlorierten Kohlenwasserstoffen oder Gemischen davon, gelöst bzw. emulgiert, das Calciumchlorid zugegeben und unter leichter Erwärmung gerührt und anschließend das Lösungsmittel wieder abgezogen Erhalten wird ein trockenes Pulver. Das Calziumchlorid wird in Mengen von 1 - 20 %, insbesondere jedoch 2 - 10 %, bezogen auf die Gewichtsmenge des 1,2-Diacyl-glycero-3-phosphocholins, zugesetzt. Bevorzugte Lösungsmittel für die 1,2-Diacyl-glycero-3-phosphocholine sind Alkohole, insbesondere Ethanol Schlämmittel sind Wasser oder Alkohole, wie Methanol oder Ethanol. Zur Trocknung eignen sich übliche Verfahren, wie Vakuum-Walzen-Trocknung, Sprühtrocknung, Gefriertrocknung. Die getrockneten, zerkleinerten 1,2-Diacylglycero-3-phosphocholin-Calziumchlorid-Mischungen können gegebenenfalls nach üblichen Verfahren zerkleinert bzw. granuliert werden. Zur Stabilisierung der Produkte werden vorteilhafterweise 0,1 - 2 Gew.-%, bezogen auf die eingesetzte 1,2-Diacylglycero-3-phosphocholin-Menge, eines Stabilisators oder Stabilisatorgemisches, wie Tocopherolacetat und/oder Ascorbinsäurepalmit, eingesetzt.

Die Herstellung der festen, oralen Arzneiformen, bestehend aus Oxicam-Derivaten und speziellen 1,2-Diacylglycero-3-phospho-cholinen, kann auch nach folgendem Verfahren erfolgen:

Pulvermischungen:
Die auf entsprechende Korngröße zerkleinerten Oxicam-Derivate und ein 1,2-Diacyl-glycero-3-phosphocholin-Calziumchlorid-Gemisch werden unter Zusatz üblicher galenischer Hilfsstoffe gemischt und zu Tabletten gepreßt oder in Kapseln abgefüllt.

Sprüheinbettungen:
Die Lösung bzw. Dispersion des Oxicam-Derivats in orgsanischen Lösungsmitteln oder Wasser werden mit einer Lösung oder Emulsion des 1,2-Diacyl-glycero-3-phosphocholins in organischen Lösungsmitteln bzw. Wasser und der Lösung des Calziumchlorids in organischen Lösungsmitteln bzw. Wasser, sowie ggfs. weiteren üblichen galenischen Hilfsstoffen gemischt und sprühgetrocknet. Die erhaltene Sprüheinbettung kann unter Zusatz weiterer galenischer Hilfsstoffe entweder zu Tabletten gepreßt oder in Kapseln, abgefüllt werden.

Unhüllungen:
Die Lösung oder Emulsion der speziellen 1,2-Diacyl-glycero-3-phosphocholine in organischen Lösungsmitteln bzw. Wasser wird mit einer Lösung des Calziumchlorids in organischen Lösungsmitteln bzw. Wasser gemischt und im Fließbett auf das vorher auf die gewünschte Korngröße zerkleinerte Oxicam-Derivats aufgetragen. Das erhaltene pulverförmige, freifließende Produkt wird unter Zusatz von galenischen Hilfsstoffen entweder zu Tabletten gepreßt oder in Kapseln abgefüllt.

Das Verhältnis Oxicam-Derivate zu 1,2-Diacyl-glycero-3-phosphocholin/Calziumchlorid kann je nach therapeutischen Erfordernissen im molaren Verhältnis von 1 : 0,5 bis 1 : 20 variieren. Vorteilhafterweise werden je Dosierung des Oxicam-Derivats 50 - 250 mg des 1,2-Diacyl-glycero-3-phosphocholin-Calzium-chlorid-Gemisches eingesetzt.

Die in den nachfolgenden Beispielen verwendeten speziellen 1,2-Diacyl-glycero-3-phosphocholine haben in den beiden Stellungen Acylgruppenreste aus den beschriebenen Fettsäurerestgemischen.

**Beispiel 1**

a.

| | |
|---|---|
| Oxicam-Derivat Pulvergemisch Piroxicam | 100 g |
| Tablettose (spezielles Aluminiumhydroxisilicat) | 800 g |
| Avicel® (microkristalline Cellulose) | 60 g |
| Aerosil® (pyrogene Kieselsäure) | 20 g |
| Natriumcarboxymethylstärke | 10 g |
| Magnesiumstearat | 10 g |

Die Stoffe werden homogen gemischt.

b.

| | |
|---|---|
| Füllmasse spezielles 1,2-Diacyl-glycero-3-phosphocholin | 9 kg |
| Polyethylenglykol 400 | 1 kg |
| Ethanol | 0,3 kg |

werden in einem Kneter bei 60° C homogen gemischt.

c. Von der unter b. beschriebenen Füllmasse werden auf einer Hartgelatinekapsel-Maschine mit Zusatzteil für Flüssigabfüllung je 160 mg der Mischung pro Hartgelatinekapsel der Größe 2 dosiert. Anschließend werden auf derselben Hartgelatinekapsel-Abfüllanlage mit Hilfe einer Abfüllstation für Pulver 100 mg der unter a. beschriebenen Pulvermischung pro Kapsel zudosiert und die Kapsel verschlossen.
Die Kapseln enthalten 50 bis 250 mg 1,2-Diacyl-glycero-3-phosphocholin sowie 10 - 20 mg nichtsteroidale Antiphlogistika.

**Beispiel 2**

a.

| | |
|---|---|
| Oxicam-Derivat Pulvermischung Piroxicam | 100 g |
| Tablettose (spezielles Aluminumhydroxisilicat) | 800 g |
| Avicel® (mikrokristalline Cellulose) | 60 g |
| Aerosil® (pyrogene Kieselsäure) | 20 g |
| Natriumcarboxymethylstärke | 10 g |
| Magnesiumstearat | 10 g |

Die Stoffe werden homogen gemischt und zu 100 mg Tabletten mit einem Durchmesser von 5 mm verpreßt.

b. Füllmasse spezielles 1,2-Diacyl-glycero-3-phosphocholin 76,5 kg
Witepsol®-Suppositorienmasse 14,3 kg
Sojaöl 9,2 kg
Ethanol 3,0 kg
werden in einem Kneter bei 60° C homogen gemischt.

c. Auf einer Hartgelatinekapselmaschine mit einer Abfüllstation für Flüssigkeiten werden 270 mg der unter b. beschriebenen Füllmasse in eine Hartgelatinekapsel der Größe 2 abgefüllt. Anschließend wird auf derselben Maschine mit Hilfe einer Abfüllstation für Tabletten ein Preßling der unter a. beschriebenen Zusammensetzung in die Kapsel eingelegt und die Kapsel verschlossen.

**Beispiel 3**

| | |
|---|---|
| Piroxicam | 100,0 g |
| spezielles 1,2-Diacyl-glycero-3-phosphocholin | 76,5 g |
| Witepsol®-Suppositorienmasse | 14,3 g |
| Sojaöl | 9,2 g |
| Ethanol | 3,0 g |

werden in einem Vorratsbehälter gemischt und unter Rühren auf ca. 40° C erwärmt. Die Lösung wird auf einer Kapselabfüllanlage des Typs Zanasi AZ 2OL durch die Dosiervorrichtung, die auf 80° C erwärmt wird, in die Kapsel gefüllt mit einer Laufgeschwindigkeit von 12.000 Kapseln je Stunde. Die Mischung verfestigt sich sofort in der Kapsel. Die Kapsel kann geschlossen und der Anlage entnommen werden.

**Beispiel 4**

| | |
|---|---|
| Piroxicam-Kapseln Piroxicam | 10,0 mg |
| Kieselsäure | 3,7 mg |
| spezielles 1,2-Diacyl-glycero-3-phosphocholin | 50,0 mg |
| Calziumchlorid | 1,3 mg |

250 g spezielles 1,2-Diacyl-glycero-phosphocholin werden in 400 ml Chloroform gelöst und mit einer Lösung von 6,875g Calziumchlorid, wasserfrei, in 70 ml Methanol gemischt. Diese Lösung wird im Fließbett auf die Pulvermischung von 50 g Piroxicam, gemahlen, < 50µm und 18,5 g Kieselsäure aufgesprüht. Das erhaltene Granulat wird über 0,5 mm abgesiebt und in Hartgelatinekapseln zu 10 mg Piroxicam abgefüllt.

**Beispiel 5**

| | |
|---|---|
| Isoxicam-Kapseln; Isoxicam | 100,0 mg |
| Kieselsäure | 5,1 mg |
| spezielles 1,2-Diacyl-glycero-3-phosphocholin | 100,0 mg |
| Calziumchlorid | 6,9 mg |

Herstellung analog Beispiel 4

**Beispiel 6**

| | |
|---|---|
| Tenoxicam-Kapseln: Tenoxicam | 10,0 mg |
| Kieselsäure | 3,5 mg |
| spezielles 1,2-Diacyl-glycero-3-phosphocholin | 50,0 mg |
| Calziumchlorid | 1,5 mg |

Herstellung analog Beispiel 4.

**Beispiel 7**

| | |
|---|---|
| Piroxicam-Tabletten: Piroxicam | 10,0 mg |
| spezielles 1,2-Diacyl-glycero-3-phosphocholin | 50,0 mg |
| Kieselsäure | 10,0 mg |
| Calziumchlorid | 4,0 mg |
| Natriumcarboxymethylstärke | 10,0 mg |
| Cellulose microcristallin | 26,0 mg |
| Magnesiumstearat | 10,0 mg |

500 g spezielles 1,2-Diacyl-glycero-3-phosphocholin werden in 1000 ml Chloroform gelöst, mit einer Lösung von 40 g Calziumchlorid, wasserfrei, in 500 ml Methanol gemischt und auf eine Pulvermischung, bestehend aus 100 g Piroxicam, gemahlen, < 50µm, und 10 g Kieselsäure im Fließbett aufgesprüht. Das erhaltene Produkt wird mit 90 g Kieselsäure, 100 g Natriumcarboxymethylstärke, 260 g Cellulose microcristallin und 100 g Magnesiumstearat in einem üblichen Pulvermischer gemischt und zu Tabletten mit 10 mg Piroxicam gepreßt.

**Beispiel 8**

| | |
|---|---|
| Piroxicam-Kapseln: Piroxicam | 20 mg |
| spezielles 1,2-Diacyl-glycero-3-phosphocholin | 100 mg |
| Kieselsäure | 40 mg |
| microkristalline Cellulose | 40 mg |

500 g spezielles 1,2-Diacyl-glycero-3-phosphocholin, zerkleinert, werden unter Zusatz von 150 g Kieselsäure kalt vermahlen. Das erhaltene Pulver wird nach Erreichen von Raumtemperatur mit 50 g Kieselsäure und 200 g Calciumhydrogenphosphat gemischt und in Kapseln zu 20 mg Piroxicam abgefüllt.

Die nach den Beispielen hergestellten pharmazeutischen Zubereitungen wurden zur Behandlung rheumatischer Erkrankungen verwendet und weisen im Vergleich zu allein in gleichen Mengen verabreichten Oxicam-Derivat Wirkstoffen erheblich geringere Nebenwirkungen durch Entzündungen im Magen-Darmtrakt und der Schädigungen der Leber auf.

Versuchsbericht zur Wirksamkeit der erfindungsgemäßen Kombination

1. Akuter Test

Für den Versuch werden männliche Ratten im Gewicht von ca. 160 g verwendet. Die Versuchstiere erhalten 3 Tage eine Brötchendiät, einen Tag vor Versuchsbeginn werden die Brötchen abgesetzt, Leitungswasser steht ad libitum weiter zur Verfügung. Die Kontroll- und Dosisgruppen bestehen aus je 10 Tieren. Die Substanzen werden p.o. in einem Volumen von 5 ml/kg KGW appliziert. 3 1/2 Stunden nach Substanzapplikation werden die Mägen entnommen und auf Ulcerationen untersucht. Die Bewertung erfolgt makroskopisch mit Hilfe von Indexzahlen. Zusätzlich wird die Ulcushäufigkeit ermittelt.

Literatur: K. Takagi, S. Okabe: Jap. J. Pharmacol.
Vol. 18, 1968, 9-18

2. Subakuter Test

Für den Versuch werden normal ernährte, männliche Ratten im Gewicht von ca. 160 g verwendet. Die Kontroll- und Dosisgruppen bestehen aus je 10 Tieren.

An drei aufeinanderfolgenden Tagen erhalten die Tiere die Prüfsubstanz p.o. in einem Volumen von 5 ml/kg KGW. 3 1/2 Stunden nach der letzten Substanzapplikation werden die Mägen entnommen und entsprechend dem Vorgehen beim akuten Test untersucht und bewertet.

**Akuter Test**

| Oxicam-Derivat (Anzahl der Tiere) | mg/kg | Ulcus-Index Oxicam-Derivat Derivat allein | Ulcus-Index Oxicam-Derivat + Phospholipid² (1 : 2 Molar) | Änderung des Ulcusindex in % |
|---|---|---|---|---|
| Piroxicam | 10,0 | 1,1 | 0,5 | - 55 |
| (n = 30) | 31,6 | 1,5 | 0,7 | - 53 |
| | 56,2 | 2,7 | 0,6 | - 78 |
| Sudoxicam | 10,0 | 0,8 | 0,4 | - 50 |
| (n = 10) | 31,6 | 1,2 | 0,6 | - 50 |
| | 100,0 | 1,5 | 0,7 | - 53 |
| Piroxicam¹) | 10,0 | 2,8 | 1,0 | - 64 |
| (n = 10) | 17,8 | 3,1 | 2,2 | - 29 |
| | 31,6 | 3,3 | 2,0 | - 39 |

1) Tiere erhielten vor dem 24 stündigen Fasten eine normale Diät

2) Phospholipid = 1,2-Diacyl-glycero-3-phosphocholine, in dem ~75 Gew.-% der Acylreste ungesättigte Fettsäurereste darstellen.

**Subakuter Test**

| Oxicam-Derivat (Anzahl der Tiere) | mg/kg | Ulcus-Index Oxicam-Derivat allein | Ulcus-Index Oxicam-Derivat + Phospholipid¹ (1 : 2 Molar) | Änderung des Ulcusindex in % |
|---|---|---|---|---|
| Piroxicam | 10,0 | 0,9 | 0,5 | - 44 |
| (n = 10) | 31,6 | 1,0 | 0,5 | - 50 |
| | 56,2 | 1,2 | 0,3 | - 75 |

1) Phospholipid: 1,2-Diacyl-glycero-3-phosphocholin, in dem 75 Gew.-% der Acylreste ungesättigte Fettsäurereste darstellen.

Vergleich der Wirksamkeit von Phosphatidylcholin mit ausschließlich gesättigten Fettsäureresten als Acylgruppen und den erfindungsgemäß zu verwendenden speziellen Phosphatidylcholinen mit Überwiegend ungesättigten Fettsäureresten.

Es wurden die Bedingungen des zuvor beschriebenen akuten und subakuten Tests angewandt.

**Akuter Test**

| Oxicam-Derivat (Anzahl der Tiere) | Dosis mg/kg | Oxicam-Derivat allein | Ulcus-Index Oxicam-Derivat + Phospholipid¹) (1 : 2 Molar) | Oxicam-Derivat + Phospholipid²) (1 : 2 Molar) | Änderung des Ulcusindex in % |
|---|---|---|---|---|---|
| Piroxicam | 10,0 | 1,1 | 0,5 | - | - 55 |
| (n = 30) | 31,6 | 1,5 | 0,7 | - | - 53 |
| | 56,2 | 2,7 | 0,6 | - | -78 |
| Piroxicam | 10,0 | 1,2 | - | 1,3 | + 8 |
| (n = 10) | 31,6 | 1,9 | - | 2,0 | + 5 |
| | 56,2 | 2,1 | - | 1,2 | - 30 |

1) Phospholipid; 1,2-Diacyl-glycero-3-phosphocholin, in dem ~75 Gew.-% der Acylreste ungesättigte Fettsäuren darstellen

2) Phospholipid: 1,2-Diacyl-glycero-3-phosphocholin, in dem die Acylreste gesättigte Fettsäuren darstellen.

Die tabellarische Darstellung der Versuchsergebnisse zeigt, daß bei niedrigen Dosierungen des Wirkstoffes Piroxicam die erwünschte Wirkung im Magen-Darmtrakt bei Phospholipiden mit ausschließlich gesättigten Fettsäureresten nicht eintritt.

Der Vergleichsversuch wurde mit Piroxicam und Diclofenac und Indomethacin im akuten Test fortgeführt (jeweils 10 Tiere).

| Verbindung | Dosis mg/kg | Wirkstoff allein | Ulcusindex gesättige Fettsäuren als Acylrest Phospholipid: Wirkstoff 2 : 1 (molar) | Äderung des Index in % |
|---|---|---|---|---|
| Piroxicam | 10 | 1.2 | 1.3 | + 8 |
| | 31.6 | 1.9 | 2.0 | + 5 |
| | 56.2 | 2.1 | 1.2 | - 30 |
| Diclofenac | 31.6 | 1.4 | 0.7 | - 50 |
| | 100 | 2.5 | 1.7 | - 32 |
| | 316 | 3.4 | 1.7 | - 50 |
| Indomethacin | 5.62 | 0.7 | 0.4 | - 43 |
| | 10 | 1.1 | 0.7 | - 36 |
| | 17.8 | 1.6 | 0.7 | - 56 |

Dieser Vergleich zeigt, daß bei anderen Wirkstoffen auch bei niedriger Dosierung mit den Phospholipiden mit gesättigten Fettsäureresten die gewünschte Wirkung eintritt, bei Piroxicam jedoch nur bei höheren Dosierungen. Der Vergleichsversuch zeigt, daß bei den Oxicamderivaten besondere Bedingungen vorliegen und spezielle Phospholipide ausgewählt werden müssen, um bei niedriger Dosierung die gewünschte Wirkung zu erreichen.

**Patentansprüche**

1. Pharmazeutisches Präparat zur therapeutischen Behandlung von rheumatischen Erkrankungen, enthaltend Phospholipide und nichtsteroidale Antiphlogistika im molaren Verhältnis 1 : 1 bis 20 : 1, dadurch gekennzeichnet, daß es

a) ein Oxicam-Derivat der allgemeinen Formel

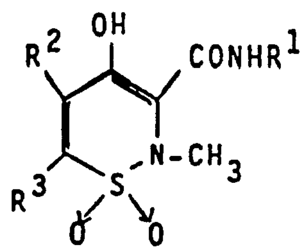

worin $R^1$ einen heterocyclischen Ring, wie z. B. Pyridin oder 5-Methyl-3-isoxazol bedeutet und $R^2$ zusammen mit $R^3$ einen ankondensierten aromatischen Ring, wie z. B. den Benzol oder Thiophenring darstellt
und
b) ein spezielles 1,2-Diacyl-glycero-3-phosphocholin, in dem 75 - 86 Gew.-% der Acylreste ungesättigte Fettsäurereste bzw. deren Gemisch mit einer Kettenlänge von 16, 18 und/oder 20 Kohlenstoffatomen bedeuten, enthält.

2. Pharmazeutisches Präparat gemäß Anspruch 1, dadurch gekennzeichnet, daß es als Oxicam-Derivat Piroxicam enthält.

3. Pharmazeutisches Präparat gemäß Anspruch 1, dadurch gekennzeichnet, daß es als Oxicam-Derivat Isoxicam enthält.

4. Pharmazeutisches Präparat gemäß Anspruch 1, dadurch gekennzeichnet, daß es als Oxicam-Derivat Tenoxicam enthält.

5. Pharmazeutisches Präparat gemäß den Ansprüchen 1 bis 4, dadurch gekennzeichnet, daß die ungesättigten Fettsäurereste des speziellen 1,2-Diacylglycero-3-phosphocholins Linolsäure- , Ölsäure-, Linolensäure- und/oder Arachidonsäurereste sind.

6. Pharmazeutisches Präparat gemäß Anspruch 5, dadurch gekennzeichnet, daß die Acylreste ein Gemisch aus Fettsäureresten aus

| | |
|---|---|
| 10 - 20 | Gew.-% Palmitinsäure |
| 3 - 5 | Gew.-% Stearinsäure |
| 8 - 12 | Gew.-% Ölsäure |
| 62 - 69,5 | Gew.-% Linolsäure |
| 4 - 7 | Gew.-% Linolensäure |

sind und wobei die Gehalte der Acylreste jeweils so ausgewählt sind, daß sie sich auf 100 Gew.-% addieren und der Anteil an ungesättigten Acylresten mindestens 75 Gew.-% und höchstens 86 Gew.-% bezogen auf Gesamtmenge Acylreste beträgt.

7. Pharmazeutisches Präparat gemäß Anspruch 5, dadurch gekennzeichnet, daß der 1-Acylrest

| | |
|---|---|
| 22 - 26 | Gew.-%[Palmitinsäure] |
| 6 - 9 | Gew.-% Stearinsäure |
| 8 - 12 | Gew.-% Ölsäure |
| 50 - 54 | Gew.-% Linolsäure |
| 4 - 6 | Gew.-% Linolensäure |

und der 2-Acylrest

| | |
|---|---|
| 1 - 2 | Gew.-% Palmitinsäure |
| 0 - 1 | Gew.-% Stearinsäure |
| 8 - 12 | Gew.-% Ölsäure |
| 75 - 85 | Gew.-% Linolsäure |
| 5 - 8 | Gew.-% Linolensäure |

als Fettsäurerestgemisch enthält und wobei sich die Gehalte der Acylreste in 1- und 2-Stellung jeweils auf 100 Gew.-% addieren und der Anteil an ungesättigten Acylresten mindestens 75 Gew.-% und höchstens 86 Gew.-% beträgt.

8. Pharmazeutisches Präparat gemäß Anspruch 5, dadurch gekennzeichnet, daß es neben dem speziellen 1,2-Diacyl-glycero-3- phosphocholin noch bis zu 20 % eines oder mehrerer
1,2-Diacyl-glycero-3-phosphate, wie z. B.
1,2-Diacyl-glycero-3-phosphoethanolamin,
1,2-Diacyl-glycero-3-phosphoinositol,
1,2Diacyl-glycero-3-phosphoserin,
1,2-Diacyl-glycero-3-phosphoglycerol
enthält, wobei die Acylreste die in Anspruch 1 angegebene Bedeutung besitzen.

9. Pharmazeutisches Präparat gemäß Anspruch 8, dadurch gekennzeichnet, daß es pro Dosiseinheit 10 - 20 mg Piroxicam und 50 - 250 mg spezielles 1,2-Diacyl-glycero-3-phosphocholin enthält.

10. Pharmazeutisches Präparat gemäß den Ansprüchen 1 - 9, dadurch gekennzeichnet, daß es pro Dosiseinheit 5 - 20 mg eines Oxicam/derivates und 10 - 500 mg spezielles 1,2-Diacyl-glycero- 3-phosphocholin neben üblichen Hilfs- und Füllstoffen enthält.

11. Verfahren zur Herstellung von pharmazeutischen Präparaten gemäß den Ansprüchen 1 - 10, dadurch gekennzeichnet, daß das Oxicam/derivat und das spezielle 1,2-Diacyl-glycero-3-phosphocholin mit üblichen Hilfs- und füllstoffen gemischt und in Kapseln abgefüllt oder zu Tabletten verpreßt wird.

12. Verfahren zur Herstellung von pharmazeutischen Präparaten gemäß den Ansprüchen 1 - 10, dadurch gekennzeichnet, daß das Oxicam/derivat in Gegenwart üblicher Hilfsstoffe in eine flüssige Masse von speziellem 1,2-Diacyl-glycero-3-phos-phocholin eingerührt und anschließend in flüssiger Form in Hartgelatinekapseln abgefüllt wird.

13. Verfahren zur Herstellung von pharmazeutischen Präparaten gemäß einem oder mehreren der Ansprüche 1 - 10, dadurch gekennzeichnet, daß das Oxicam/derivat und das spezielle 1,2-Diacyl- glycero-3-phosphocholin zusammen in Wasser oder in einem organischen Lösungsmittel gelöst oder emulgiert wird, Calziumchlorid, in Wasser oder einem Alkohol gelöst oder aufgeschlämmt, zugegeben wird, die Lösungsmittel abgezogen werden, das erhaltene Produkt nach üblichen Verfahren getrocknet, ggfs. gemahlen, und nach üblichen Verfahren zu pharmazeutischen Zubereitungen verarbeitet wird.

14. Verfahren zur Herstellung von pharmazeutischen Präparaten gemäß einem oder mehreren der Ansprüche 1 - 10, dadurch gekennzeichnet, daß das Oxicam/derivat auf die gewünschte Korngröße zerkleinert, mit einem pulvrigen, speziellen 1,2-Diacylglycero-3-phosphocholin-Calziumchlorid-Gemisch gemischt und zu üblichen Arzneiformen, wie Tabletten und Kapseln, verarbeitet wird.

15. Verfahren zur Herstellung von pharmazeutischen Präparaten gemäß einem oder mehreren der Ansprüche 1 - 10, dadurch gekennzeichnet, daß die Lösung bzw. Dispersion des Oxicam/derivats in organischen Lösungsmitteln oder Wasser mit einer Lösung oder Emulsion des speziellen 1,2-Diacyl-glycero-3-phosphocholins in organischen Lösungsmitteln bzw. Wasser und einer Lösung von Calziumchlorid in organischen Lösungsmitteln bzw. Wasser, sowie ggfs. mit weiteren üblichen galenischen Hilfsstoffen gemischt und sprühgetrocknet, und die erhaltene Sprüheinbettung ggfs. unter Zusatz weiterer galenischer Hilfsstoffe entweder zu Tabletten verpreßt oder in Kapseln abgefüllt wird.

16. Verfahren zur Herstellung von pharmazeutischen Präparaten gemäß einem oder mehreren der Ansprüche 1 - 10, dadurch gekennzeichnet, daß eine Lösung oder Emulsion des speziellen 1,2-Diacyl-glycero-3-phosphocholines in organischen Lösungsmitteln bzw. Wasser mit einer Lösung von Calziumchlorid in organischen Lösungsmitteln bzw. Wasser gemischt und im Fließbett auf das vorher auf die gewünschte Korngröße zerkleinerte Oxicam/derivat aufgebracht wird.

**Claims**

1. Pharmaceutical preparation for the therapeutic treatment of rheumatic diseases comprising phospholipids and nonsteroidal antiphlogistics at a molar ratio of 1 : 1 to 20 : 1,
characterized in that,
it contains

(a) an oxicam derivative of the general formula

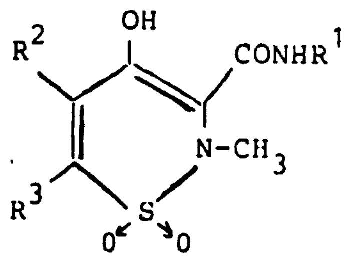

in which $R^1$ signifies a heterocyclic ring such as pyridine or 5-methyl-3-isoxazole and $R^2$ together with $R^3$ represents a fused-on aromatic ring, such as the benzene or thiophene ring,
and

(b) a special 1,2-diacyl glycero-3-phosphocholine, in which 75 - 86 % by weight of the acyl radical comprise unsaturated fatty acid radicals or mixtures thereof with a chain length of 16, 18 and/or 20 carbon atoms.

2. Pharmaceutical preparation according to claim 1,
characterized in that,
it contains piroxicam as the oxicam derivative.

3. Pharmaceutical preparation according to claim 1,
characterized in that,
it contains Isoxicam as the oxicam derivative.

4. Pharmaceutical preparation according to claim 1,
characterized in that,
it contains Tenoxicam as the oxicam derivative.

5. Pharmaceutical preparation according to claims 1 - 4,
characterized in that,
the unsaturated fatty acid radicals of the special 1.2-diacyl glycero-3-phosphocholines are radicals of linoleic acid, oleic acid, linolenic acid and/or arachidonic acid.

6. Pharmaceutical preparation according to claim 5
characterized in that,
the acyl radicals are a mixture of fatty acid radicals consisting of

| | |
|---|---|
| 10 - 20 % | by weight palmitic acid |
| 3 - 5 % | by weight stearic acid |
| 8 - 12 % | by weight oleic acid |
| 62 - 69.5 % | by weight I inoleic acid and |
| 4 - 7 % | by weight linolenic acid |

and where the content of the acyl radicals at any one time is selected such that they totalize 100 % by weight and the portion of unsaturated acyl radicals is at least 75 % by weight and at the most 86 % by weight of the total amount of acyl radicals.

7. Pharmaceutical preparation according to claim 5,
characterized in that,
the 1-acyl radical contains as a mixture of fatty acid radicals

| | |
|---|---|
| 22 - 26 % | by weight palmitic acid |
| 6 - 9 % | by weight stearic acid |
| 8 - 12 % | by weight linoleic acid |
| 50 - 54 % | by weight linoleic acid |
| 5 - 6 % | by weight linolenic acid, |

and the 2-acyl radical similarly contains

| | |
|---|---|
| 1 - 2 % | by weight palmitic acid |
| 0 - 12 % | by weight stearic acid |
| 8 - 12 % | by weight oleic acid |
| 75 - 85 % | by weight linoleic acid |
| 5 - 8 % | by weight molenic acid, |

whereby the contents of 1- and 2-acyl radicals totalize 100 % by weight and the proportion of unsaturated acyl radicals is at least 75 % by weight and at the most 86 % by weight.

8. Pharmaceutical preparation according to claim 5,
characterized in that,
apart from the special 1.2-diacyl glycero-3-phosphocholine, it also contains up to 20 % of one or more 1.2-diacyl glycero -3-phosphates, such as
1.2-diacyl glycero-3-phosphoethanolamine,
1.2-diacyl glycero-3-phosphoinositol,
1.2-diacyl glycero-3-phosphoserine and
1.2-diacyl glycero-3-phosphoglycerol,
while the acyl radicals have the meaning given in claim 1.

9. Pharmaceutical preparation according to claim 8,
characterized in that,
it contains 10 - 20 mg Piroxicam and 50 - 250 mg of special 1.2-diacyl glycero-3-phosphocholine per unit dose.

10. Pharmaceutical preparation according to claims 1 - 9,
characterized in that,
it contains 5 - 20 mg of an oxicam derivative and 10 - 500 mg of special 1.2-diacyl glycero-3-phosphocholine per unit dose together with conventional adjuvants and fillers.

11. Method for the manufacture of pharmaceutical preparations according to claims 1 - 10,
characterized in that,
the oxicam derivative and the special 1.2-diacyl glycero-3-phosphocholine are mixed with conventional adjuvants and fillers and filled into capsules or compressed into tablets.

12. Method for the manufacture of pharmaceutical preparations according to claims 1 - 10,
characterized in that,
the oxicam derivative is stirred into a fluid mass of special 1.2-diacyl glycero-3-phosphochol ine in the presence of conventional adjuvants and then filled into hard gelatine capsules in a fluid form..

13. Method for the manufacture of pharmaceutical preparations according to one or more of claims 1 - 10,
characterized in that,
the oxicam derivative and the special 1.2-diacyl glycero-3-phosphocholine are dissolved or emulsified together in water or an organic solvent, calcium chloride dissolved or slurried in water or an alcohol is added, the solvent is taken off, the resulting product is dried by a conventional method, milled if desired and processed into pharmaceutical preparations by conventional methods.

14. Method for the manufacture of pharmaceutical preparations according to one or more of claims 1 - 10,
characterized in that,
the oxicam derivative is comminuted to the desired particle size, mixed with a pulverulent special 1.2-diacyl glycero-3-phosphocholine/calcium chloride mixture and processed into conventional pharmaceutical forms such as tablets and capsules.

15. Method for the manufacture of pharmaceutical preparations according to one or more of claims 1 - 10,
characterized in that,
the solution resp. dispersion of the oxicam derivative in organic solvents or water is mixed with a solution or emulsion of the special 1.2-diacyl glycero-3-phsphocholine in organic solvents or water and a solution of calcium chloride in organic solvents, resp. water, as well as, if desired, with further conventional galenical adjuvants, and spray dried, and the resulting spray-dried product is, if desired, by adding further galenical adjuvants, either compressed into tablets or filled into capsules.

16. Method for the manufacture of pharmaceutical preparations according to one or more of claims 1 - 10,
characterized in that,
a solution or emulsion of the special 1.2-diacyl glycero-3-phosphocholine in organic solvents or water is mixed with a solution of calcium chloride in organic solvents resp. water and applied in a fluidized bed to the oxicam derivative which has previously been comminuted to the desired particle size.

**Revendications**

1. Préparation pharmaceutique pour le traitement thérapeutique de maladies rhumatismales, contenant des phospholipides et des anti-inflammatoires non stéroïdes dans le rapport molaire 1 : 1 et 20 : 1, caractérisée en ce qu'elle contient

a) un dérivé de l'oxicam de la formule générale

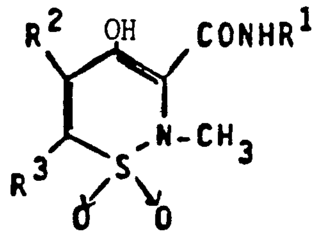

où $R^1$ signifie un composé hétérocyclique comme par exemple la pyridine ou le 5-méthyl-3-isoxazol, où $R^2$ forme avec $R^3$ , un composé cyclique aromatique condensé, comme par exemple le composé cyclique benzène ou thiophène

et

b) une 1,2-diacyl-glycéro-3-phosphocholine spéciale dans laquelle les radicaux acyle, en proportion de 75 à 86 % en poids, signifient des radicaux d'acides gras non saturés ou leurs mélanges avec une longueur de chaine de 16, 18 et/ou 20 atomes de carbone.

2. Préparation pharmaceutiques selon la revendication 1,
caractérisée en ce qu'elle contient comme dérivé de l'oxicam, du piroxicam.

3. Préparation pharmaceutique selon la revendication 1,
caractérisée en ce qu'elle contient comme dérivé de l'oxicam, de l'isoxicam.

4. Préparation pharmaceutique selon la revendication 1,
caractérisée en ce qu'elle contient comme dérivé du l'oxicam, du ténoxicam.

5. Préparation pharmaceutique selon les revendications 1 à 4,
caractérisée en ce que les radicaux acides gras non saturés de la 1,2 diacylglycéro-3-phosphocholine spéciale sont les radicaux acide linoléique, acide oléique, acide linolénique et/ou acide arachidonique.

6. Préparation pharmaceutique selon la revendication 5,
caractérisée en ce que les radicaux acyle sont un mélange de radicaux acides gras constitués de

| | |
|---|---|
| 10 - 20 | % en poids acide palmitique |
| 3 - 5 | % en poids acide stéarique |
| 8 - 12 | % en poids acide oléique |
| 62 - 69,5 | % en poids acide linoléique |
| 4 - 7 | % en poids acide linolénique |

les teneurs des radicaux acyle étant respectivement choisis de façon à obtenir 100, en poids par addition et de façon que la proportions de radicaux acyle non saturés atteigne au moins 75 % en poids et au plus 86 % en poids, rapporté à la quantité totale de radicaux acyle.

7. Préparation pharmaceutique selon la revendication 5,
caractérisée en ce que le radical acyle en position 1 contient comme mélange de radicaux d'acides gras

| | |
|---|---|
| 22 - 26 | % en poids acide palmitique |
| 6 - 9 | % en poids acide stéarique |
| 8 - 12 | % en poids acide oléique |
| 50 - 54 | % en poids acide linoléique |
| 5 - 6 | % en poids acide linolénique |

et le radical acyle en position 2

| | |
|---|---|
| 1 - 2 | % en poids acide palmitique |
| 0 - 1 | % en poids acide stéarique |
| 8 - 12 | % en poids acide oléique |
| 75 - 85 | % en poids linoléique |
| 5 - 8 | % en poids acide linolénique |

et étant précisé que les teneurs des radicaux acyle en position 1 et en position 2 donnent respectivement au total, par addition, 100 % en poids, et que la proportion en radicaux acyle non saturés atteint au moins 75 % en poids et au plus 86 % en poids.

8. Préparation pharmaceutique selon la revendication 5, caractérisée en ce qu'à côté de la 1,2 diacyl-glycéro-3- phosphocholine spéciale, elle contient encore jusqu'à 20 % d'un ou plusieurs
1,2 diacyl-glycéro-3-phosphates, comme par exemple
1,2 diacyl-glycéro-3-phosphoéthanolamine
1,2 diacyl-glycéro-3-phosphoinositol
1,2 diacyl-glycéro-3-phosphosérine
1,2 diacyl-glycérino-3-phosphoglycérol
étant précisé que les radicaux acyle ont la signification indiquée dans la revendication 1.

9. Préparation pharmaceutique selon la revendication 8,
caractérisée en ce que, par dose, elle contient 10 - 20 mg de piroxicam et 50 - 250 mg de 1,2 diacyl-glycéro-3-phosphocholine spéciale.

10. Préparation pharmaceutique selon les revendications 1 - 9, caractérisée en ce que, par dose, elle contient 50 - 20 mg d'un dérivé de l'oxicam et 10 - 500 mg de 1,2 diacyl-glycéro-3-phosphocholine en plus des adjuvants et des charges habituelles.

11. Procédé de fabrication d'une préparation pharmaceutique selon les revendications 1 - 10, caractérisé en ce que l'on mélange le dérivé de l'oxicam et la 1,2 diacyl-glycéro-3-phosphocholine spéciale avec les adjuvants et les charges habituelles, qu'on le met en capsules ou qu'on le presse en tablettes.

12. Procédé d'une préparation pharmaceutique selon les revendications 1 - 10, caractérisé en ce que l'on mélange le dérivé de l'oxicam en présence des adjuvants habituels dans une masse fluide de 1,2 diacyl-glycéro-3-phosphocholine spéciale et qu'ensuite on le met, sous forme fluide, dans des capsules de gélatine dure.

13. Procédé de fabrication de préparations pharmaceutiques selon une ou plusieurs des revendications 1 - 10, caractérisé en ce que l'on dissout ou que l'on émulsifie le dérivé de l'oxicam et la 1,2 diacyl-glycéro-3-phosphocholine spéciale dans l'eau ou dans un solvant organique, que l'on dissout du chlorure de calcium dans l'eau ou dans un alcool, ou qu'on en fait une bouillie, qu'on l'y ajoute, qu'on extrait le solvant, qu'on sèche le produit obtenu par un procédé habituel, éventuellement qu'on le broie et qu'on le conditionne selon les procédés habituels pour préparations pharmaceutiques.

14. Procédé de fabrication de préparations pharmaceutiques selon une ou plusieurs des revendications 1 - 20, caractérisé en ce que l'on broie le dérivé de l'oxicam à la granulométrie désirée, qu'on le mélange avec un mélange pulvérulent de 1,2 diacyl-glycéro-3-phosphocholine spéciale et de chlorure de calcium et qu'on le conditionne sous une

forme habituelle des médicaments, par exemple tablettes et capsules

15. Procédé de préparations pharmaceutiques selon une ou plusieurs des revendications 1-10, caractérisé en ce que l'on mélange la solution et la dispersion du dérivé de l'oxicam dans des solvants organiques ou dans de l'eau avec une solution ou une émulsion de la 1,2 diacyl-glycéro-3-phosphochline spéciale dans les solvants organiques ou dans de l'eau et avec une solution de chlorure de calcium dans les solvants organiques ou dans de l'eau, ainsi qu'éventuellement avec d'autres adjuvants galéniques habituels, et qu on les fait sécher par pulvérisation, puis que soit on comprime l'enrobage ainsi obtenu, éventuellement en y ajoutant d'autres adjuvants galéniques, pour en faire des tablettes, soit qu'on le met en capsules.

16. Procédé de fabrication de préparations pharmaceutiques selon l'une ou plusieurs des revendications 1 - 10, caractérisé en ce que l'on mélange une solution ou une émulsion de la 1,2 diacyl-glycéro-3-phosphocholine spéciale dans des solvants organiques ou dans de l'eau avec une solution de chlorure de calcium dans des solvants organiques ou dans de l'eau, puis que l'on applique le mélange en lit fluide sur le dérivé d'oxicam préalablement broyé à la granulométrie désirée.